(19) Europäisches Patentamt
European Patent Office
Office européen des brevets

(11) **EP 3 272 290 B1**

(12) **EUROPEAN PATENT SPECIFICATION**

(45) Date of publication and mention
of the grant of the patent:
**10.11.2021 Bulletin 2021/45**

(51) Int Cl.:
*A61B 8/00* (2006.01)   *A61B 8/13* (2006.01)
*A61B 8/08* (2006.01)   *G06T 5/00* (2006.01)
*G06T 5/50* (2006.01)

(21) Application number: **15885652.6**

(22) Date of filing: **24.07.2015**

(86) International application number:
**PCT/KR2015/007733**

(87) International publication number:
**WO 2016/148350 (22.09.2016 Gazette 2016/38)**

(54) **DEVICE AND METHOD FOR RECONSTRUCTING MEDICAL IMAGE**

VORRICHTUNG UND VERFAHREN ZUR REKONSTRUKTION EINES MEDIZINISCHEN BILDES

DISPOSITIF ET PROCÉDÉ POUR LA RECONSTRUCTION D'IMAGES MÉDICALES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.03.2015 KR 20150037652**

(43) Date of publication of application:
**24.01.2018 Bulletin 2018/04**

(73) Proprietors:
• **Vatech Co., Ltd.**
**Hwaseong-si, Gyeonggi-do 445-170 (KR)**
• **Vatech Ewoo Holdings Co., Ltd.**
**Hwaseong-si, Gyeonggi-do 445-170 (KR)**

(72) Inventors:
• **IM, Se Yeol**
**Hwaseong-si**
**Gyeonggi-do 445-170 (KR)**
• **KIM, Tae Woo**
**Hwaseong-si**
**Gyeonggi-do 445-170 (KR)**
• **SEO, Dong Wan**
**Hwaseong-si**
**Gyeonggi-do 445-170 (KR)**
• **CHOI, Sung Il**
**Hwaseong-si**
**Gyeonggi-do 445-170 (KR)**

• **HAN, Tae Hee**
**Hwaseong-si**
**Gyeonggi-do 445-170 (KR)**

(74) Representative: **Markfort, Iris-Anne Lucie**
**Lorenz & Kollegen**
**Patentanwälte Partnerschaftsgesellschaft mbB**
**Alte Ulmer Straße 2**
**89522 Heidenheim (DE)**

(56) References cited:
JP-A- 2007 130 240      KR-A- 20100 070 822
US-A1- 2013 022 252     US-A1- 2013 235 036
US-A1- 2015 036 790

• FARMAN ET AL: "Development of imaging
selection criteria and procedures should precede
cephalometric assessment with cone-beam
computed tomography", AMERICAN JOURNAL
OF ORTHODONTICS AND DENTOFACIAL
ORTHOPE, MOSBY, ST. LOUIS, MO, US, vol. 130,
no. 2, 1 August 2006 (2006-08-01), pages 257-265,
XP005592973, ISSN: 0889-5406, DOI:
10.1016/J.AJODO.2005.10.021
• V KUMAR: "Comparison of conventional and
cone beam CT synthesized cephalograms",
MASTER THESIS, no. 5, 1 January 2007
(2007-01-01), pages 1-69, XP055513567,
• None

# EP 3 272 290 B1

**Description**

Technical Field

The present invention relates to a device and method of reconstructing a medical cephalometric image, and a computer readable recording medium for implementing the same as defined in the independent claims.

Background Art

In general, 3D medical images such as a CT image, an ultrasonic image, etc. are capable of generally and clearly examining an examinee. However, in order to observe an internal structure of the examinee in more detail, it may be more effective to use a two-dimensional medical image rather than a three-dimensional medical image. Accordingly, when a 3D medical image and a 2D medical image are displayed at the same time, a user may generally and clearly observe the examinee at the same time.

A conventional example of displaying a 3D medical image and a 2D medical image is disclosed in Korean Patent Application No. 10-2013-0138612 (Publication Date: 19 December 2013), and is as below.

A conventional device for displaying a 3D medical image and a 2D medical image includes: a 3D image obtaining unit obtaining a 3D medical image of an examinee; a cross-section selecting unit selecting at least one cross-section of the examinee based on an external input for the obtained 3D medical image; a 2D image obtaining unit obtaining a 2D medical image by scanning the examinee corresponding to the selected at least one cross section; and a displaying unit displaying the 3D medical image and the 2D medical image.

Herein, the cross-section selection unit includes: a window generator generating at least one window on which the obtained 3D medical image is disposed; a window controller moving the at least one generated window in the 3D medical image; and an additional cross-section selector additionally selecting at least one cross-section adjacent to the at least one selected cross-section.

In addition, the 2D image obtaining unit includes: a first image obtainer obtaining at least one first 2D medical image by scanning the examinee according to the at least one selected cross-section; a second image obtainer obtaining at least one second 2D medical image by scanning the examinee according to a cross-section adjacent to the at least one selected cross-section; and a combined image obtainer obtaining at least one combine 2D medical image by combining the at least one first 2D medical image and the at least one second 2D medical image.

In the conventional technique, at least one combined 2D medical image may be obtained by respectively obtaining first and second 2D medical images according to at least one cross-section and at least one cross-section adjacent to the cross-section, and by combining the first and second 2D medical images. However, the combined 2D medical image obtained in this manner differs from an actually radiographed 2D medical image.

This is because, since an X-ray beam or ultrasonic beam has a feature that beams thereof are irradiated in a radial shape, magnification degrees between 2D medical images corresponding to different cross-sections spaced apart by a certain distance or more on the beam path are different. Accordingly, when a 2D medical image is obtained by adding medical images within a selected area (range) using a simple image combining method such as method used in conventional technique, there is a problem that an inaccurate 2D medical image is obtained.

Meanwhile, when radiographing, in many cases, radiographing may be performed while a patient (examinee) is not posing accurately for the radiographing according to a skill of a radiographer.

Thus, a CT image is radiographed in a state in which the patient is posing in a wrong posture may not prevent a situation in which bilateral symmetry and facial skeleton structures appear to be misaligned.

Accordingly, when a CT image obtained in a state in which a posture of the patient is not accurately when radiographing is displayed through a medical diagnosing 3D viewer, there is a problem the CT image looks distorted.

Reference is also made to FARMAN ET AL: "Development of imaging selection criteria and procedures should precede computed tomographie", American Journal of Orthondontics and Dentofacial Orthope, Mosby, St. Louis, MO, US, vol. 130, no. 2, 1°August 2006 (2006-08-01), pages 257-265, XP005592973, ISSN: 0889-5406, DOI: 10.1016/J.AJODO.2005.10.021.

Disclosure

Technical Problem

Accordingly, the present invention has been made to solve the above-mentioned problems occurring in the prior art.

Accordingly, an object of the present invention is to provide a device and method of reconstructing a medical image for generating a 2D medical image using an automatic reconstruction method by correcting an error generated due to an inaccurate posture of a patient when radiographing by using pre-radiographed 3D medical images so that a corresponding image is correctly positioned at a 3D space without additionally radiographing a 2D medical image, a cephalometric image, and a computer readable recording medium for implementing the same.

The objectives of the present invention are not limited to those mentioned above. Other objectives and advantages of the present invention which are not disclosed will be understood from the following description, and will be apparent with reference to the embodiments of the present invention. Also, it is obvious to those skilled in the art that the objectives and advantages of the present invention will be realized by the means as claimed.

**Technical Solution**

A device of the present invention for accomplishing the above object includes the features of claim 1.

In addition, a method of the present invention for accomplishing the above object includes the features of claim 4.

**Advantageous Effects**

According to the present invention as described above, a cephalometric image is generated by correcting an error generated due to an accurate posture of a patient when radiographing by using a pre-radiographed 3D medical image so that a corresponding image is correctly positioned at a 3D space, and by generating a 2D medical image using an automatic reconstruction method, thus there is no need for additionally radiographing a cephalometric image, and an exposure dose of an examinee (patient) may be reduced by removing additional exposure.

In addition, in the present invention, a positional accuracy for each cross-sectional image may be improved by correcting an error generated by an inaccurate posture of a patient when radiographing. Accordingly, it is possible to make an accurate medical diagnosis.

In addition, in the present invention, a misalignment of a facial skeleton structure in an image may be prevented by applying a CT geometry correction by using CT information when automatically generating a cephalometric image using an automatic reconstruction method. Accordingly, a positional accuracy for each cross-sectional image and an enlarged image may be improved.

In addition, in the present invention, when implementing a cephalometric image radiograph device, attaching of an arm that guarantees a certain magnification degree or more

may be unnecessary. Accordingly, a size of the medical device and an installation space thereof may be reduced.

Description of Drawings

FIG. 1 is a configuration diagram of a medical image reconstruction device according to an embodiment of the present invention.

FIG. 2 is a detailed configuration diagram of a geometry correction unit according to an embodiment of the present invention.

FIG. 3 is a detailed configuration diagram of a correction angle extractor according to an embodiment of the present invention.

FIG. 4 is a detailed configuration diagram of a rotation angle count map extractor according to an embodiment of the present invention.

FIG. 5 is a flowchart of a medical image reconstruction method according to an embodiment of the present invention.

FIG. 6 is a detailed flowchart of a geometry correction process according to an embodiment of the present invention.

FIG. 7 is a detailed flowchart of a correction angle extraction process according to an embodiment of the present invention.

FIG. 8 is a detailed flowchart of a rotation angle count map extraction process according to an embodiment of the present invention.

**Mode for Invention**

The above and other objects, features, and advantages of the present invention can be appreciated by the following description and will be understood more clearly by an embodiment of the present invention. In addition, it will be appreciated that the objects and advantages of the present invention will be easily realized by means shown in the appended patent claims.

Through the whole document, the term "connected to" or "coupled to" that is used to designate a connection or coupling of one element to another element includes both a case that an element is "directly connected or coupled to" another element and a case that an element is "electronically connected or coupled to" another element via still another element. Also, when a part may "include or comprise" a certain constituent element, unless specified otherwise, it may not be construed to exclude another constituent element but may be construed to further include other constituent elements.

Hereinafter, preferred embodiments of the present invention will be described in detail with reference to the accompanying drawings.

First, technical points according to the embodiment of the present invention will be summarized as follows.

In the embodiment of the present invention, a cephalometric image is generated by an automatic reconstruction method by applying a CT geometry correction to CT data so that a CT image is correctly positioned at a three-dimensional space, and by using the corrected CT image. Herein, in the embodiment of the present invention, the cephalometric image is generated by reconstructing image data of a specific section in a sagittal or coronal cross-sectional direction of a CT image based on an X-ray irradiation path.

In other words, in the embodiment of the present invention, first, in order to correct an error generated by an inaccurate posture of a patient while radiographing, a positional error is corrected by applying a CT geometry correction algorithm. Herein, an optimized position is calculated by using a feature value of a region of interest (ROI) in the CT image. Then, in order to generate a cephalometric image in a CT image, an automatic reconstruction method algorithm is applied. Herein, the cephalometric image is generated by performing a summation of image data of a designated section in a sagittal or coronal cross-sectional direction based on an X-ray irradiation path.

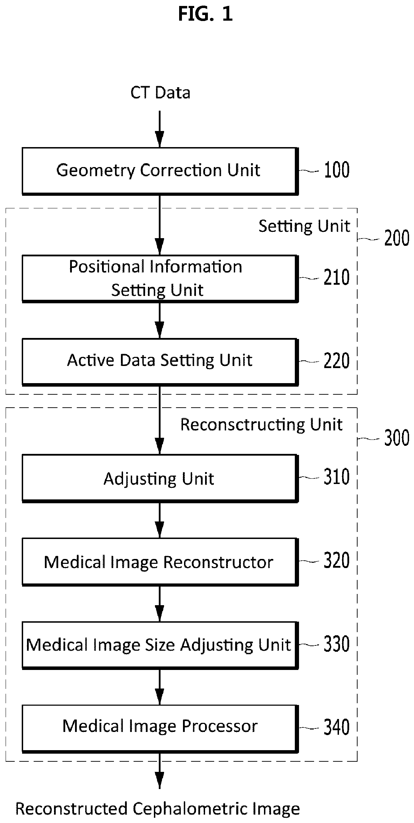

FIG. 1 is a configuration diagram of a medical image reconstruction device according to an embodiment of the present invention.

As shown in FIG. 1, the medical image reconstruction device according to the embodiment of the present invention includes: a geometry correction unit 100 correcting so that a CT image is correctly positioned at a three-dimensional space by applying a CT geometry correction to CT data; a setting unit 200 setting an active image data section that will be used for reconstructing a cephalometric image; and a reconstructing unit 300 reconstructing a two-dimensional (2D) medical image by performing a summation of "image data of the CT image corrected in the geometry correction unit 100" within the active image data section set in the setting unit 200 based on an X-ray irradiation path.

Next, detailed description of respective components will be described as follows.

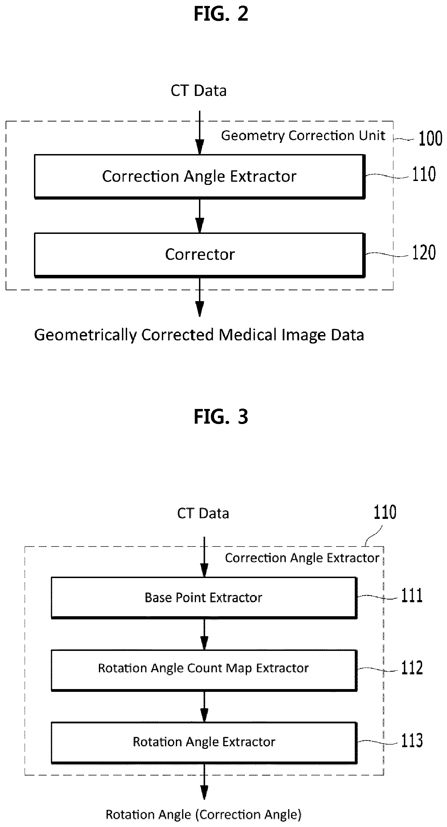

First, the geometry correction unit 100 performs a CT geometry correction to correct an error generated by an inaccurate posture of a patient when radiographing. Herein, an image after applying the CT geometry correction has a form in which a face of the patient is facing the front. Bilaterally symmetric organ chin, eyes, etc., or other feature points maintain a horizontal level. And the image is a rearranged so that the head is not bent too much or is held. The geometry correction unit 100 will be described in more detail with reference to FIGS. 2 to 4.

FIG. 2 is a detailed configuration diagram of the geometry correction unit 100 according to the embodiment of the present invention.

As shown in FIG. 2, the geometry correction unit 100 according to the embodiment of the present invention includes: a correction angle extractor 110 receiving CT data, and extracting a correction angle for each cross-sectional direction for a positional correction of the CT data; a corrector 120 performing a geometry correction by rotating a CT image by using the correction angle for each cross-sectional direction extracted by the correction angle extractor 110.

Next, detailed descriptions of respective components will be described as follows.

First, the CT radiograph device (not shown in the figure) radiographs, for example, a head of an examinee, and transmits CT data to a geometry correction device of medical image data.

Then, the correction angle extractor 110 receives the CT data from the CT radiograph device, and extracts a correction angle for each cross-sectional direction (coronal, sagittal, axial) of a multi planar reconstruction (MPR) for a positional correction of the CT data. Herein, in order to extract the correction angle for each cross-sectional direction of the MPR, a sub-routine (correction angle extractor of FIG. 3) is performed. Herein, the MPR means reconstructing a number of cross sectional images from a three-dimensional image. Coronal, sagittal, and axial images are related with each cross section of X, Y, and Z axis of the three-dimensional image.

In addition, the corrector 120 performs a geometry correction by rotating the CT image by using a rotation angle (correction angle) extracted for each cross-sectional direction in the correction angle extractor 110. Herein, the rotation angle (correction angle) may be calculated as three parameters according to coronal, sagittal, and axial directions.

FIG. 3 is a detailed configuration diagram of the correction angle extractor 110 according to the embodiment of the present invention.

As shown in FIG. 3, the correction angle extractor 110 according to the embodiment of the present invention includes: a base point extractor 111 extracting base point in the CT data; a rotation angle count map extractor 112 extracting a count map for each rotation angle by using image data of an ROI including the base point extracted in the base point extractor 111; and a rotation angle extractor 113 extracting a rotation angle of the minimum count map among the count map for each rotation angle extracted in the rotation angle count map extractor 112.

Next, detailed descriptions of respective components will be described as follows.

First, the base point extractor 111 receives the CT data from the CT radiograph device, and extracts any of the base point which may be extracted within the CT data. Herein, as the base point, jawline information, teeth arrangement information, ear plug information, temporo-mandibular joint (TMJ) information, eye positional information, etc. may be included.

For example, when jawline information of a lower jaw (coordinate at three-dimensional space) is used as the base point, an overlapped image is obtained by overlapping an axial cross-sectional image of a designated area within the CT image. When obtaining the overlapped image, an arbitrary threshold value that will be described later is used for extracting the jawline information of the lower jaw. Herein, the Formula for generating the overlapped image is as the [Formula 1] below.

[Formula 1]

$$g(x,y) = \sum_{z=zs}^{ze} \sum_{y=0}^{h-1} \sum_{x=0}^{w-1} f(x,y,z)$$

Herein, x, y, and z means positional values of X, Y, and Z axis, w is a horizontal size of a cross-sectional image, and h is a vertical size of a cross-sectional image. Zs is a start point that is set in an Z axis to overlap an image, and ze is an end point that is set in the Z axis to overlap the image. Starting point and stopping point values for overlapping a CT image may vary since the values may be changed according to a characteristic of a patient, and may be used by optimizing through an experiment. The corresponding example is as the [Formula 2] below.

[Formula 2]

$$zs = z - 20$$
$$ze = zs - (z * 0.45)$$

In addition, the Formula of an overlapped image generation condition is as the [Formula 3] below.

[Formula 3]

$$f(x,y,z) = \{g(x,y) + 1, \quad t1 < I(x,y,z) < t2\}$$

Herein, I(x,y,z) means each pixel value of the CT image, when a designated threshold value is satisfied, a value of an overlapped image g(x,y) is increased. A designated threshold value t1 is 1000, and t2 is 2000. Herein, the threshold values t1 and t2 may be changed since the values may be changed according to the CT radiograph device or a radiograph condition, and may be used by optimizing through an experiment

In addition, a central area of the lower jawline is calculated by using overlapped image data. Image data of the central area of the lower jawline within the designated section of the axial cross-sectional image in the CT image is extracted. The extracted image data uses a coordinate of a cross-section that satisfies a condition that will be described later as the central area of the lower jawline. The Formula h(x,y) for detecting the central area of the lower jawline is as the [Formula 4] below.

[Formula 4]

$$h(x,y) = \sum_{z=zs}^{ze} \sum_{y=by-sy}^{by+sy} \sum_{x=bx-sx}^{bx+sx} j(x,y,z)$$

Herein, bx and by are a central coordinate of the designated section for detecting the central area of the lower jawline within the overlapped image, sx and sy are size values of corresponding sections. Herein, the Formula for setting the corresponding section is as the [Formula 5] below.

[Formula 5]

$$bx = \frac{1}{2}\left( \sum_{y=0}^{h-1} \sum_{x=0}^{w-1} X(x,y) + \sum_{y=0}^{h-1} \sum_{x=w-1}^{0} X(x,y) \right)$$

$$by = \sum_{y=0}^{h-1} \sum_{x=0}^{w-1} Y(x,y)$$

In addition, the Formulas X(x,y) and Y(x,y) for detecting an X-axial central coordinate of the designated section is as the [Formula 6] below.

[Formula 6]

$$X(x,y) = \{x, \qquad g(x,y) > t\}$$
$$Y(x,y) = \{y, \qquad bx \neq 0\}$$

In order to set a valid point within the overlapped image, an arbitrary threshold value t is used as a base. For example, the threshold value t is 40. Herein, a value of the threshold value t may be changed since the value may be changed according to an image characteristic, and may be used by optimizing through an experiment.

For example, all of area size values sx and sy for setting the section based on the detected central coordinate are 100. Herein, the area size values may be changed according to an image characteristic, and may be used by optimizing through an experiment.

Finally, the Formula j(x,y,z) for detecting jawline information of the lower jaw within the set section is as the [Formula 7] below.

[Formula 7]

$$j(x,y,z) = \{z, \qquad TC = m * m\}$$

Herein, m means a mask size, a total number TC in which a pixel value satisfies the threshold values t1 and t2 by retrieving a mask area adjacent to each pixel within the designated area is calculated as the [Formula 8] below.

[Formula 8]

$$TC = \sum_{my=y-m/2}^{y+m/2} \sum_{mx=x-m/2}^{x+m/2} k(mx,my)$$

$$k(mx,my) = \{TC + 1, \qquad t1 < I(x,y,z) < t2\}$$

$$t1 = 1000$$
$$t2 = 2000$$

Herein, when a TC value is equal to a size of the mask area, a corresponding positional point is used as a positional coordinate of the lower jawline.

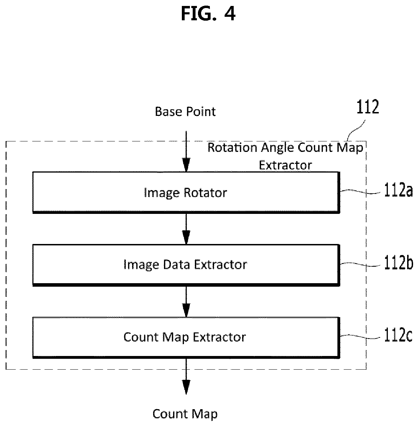

In addition, the rotation angle count map extractor 112 extracts a count map for each preset rotation angle by using image data of the ROI extracted based on the base point extracted in the base point extractor 111. Herein, a sub-routine (rotation angle count map extractor of FIG. 4) is performed for each rotation angle to extract the count map for each rotation angle.

In addition, the rotation angle extractor 113 extracts a rotation angle of the count map having the fewest pixel numbers (in other words, the minimum count map) by measuring a number of valid pixels of the count map extracted for each rotation angle in the rotation angle count map extractor 112.

FIG. 4 is a detailed configuration diagram of the rotation angle count map extractor 112 according to the embodiment of the present invention.

As shown in FIG. 4, the rotation angle count map extractor 112 according to the embodiment of the present invention includes: an image rotator 112a rotating the CT image for each preset rotation angle; an image data extractor 112b extracting image data of an ROI based on the base point extracted in the base point extractor 111 from the CT image rotated in the image rotator 112a; and a count map extractor 112c extracting the count map by using the image data of the ROI extract in the image data extractor 112b .

Next, detailed descriptions of respective components will be described as follows.

First, the image rotator 112a repeatedly rotates the CT image by a pre-designated (set) rotation angle section with a preset arbitrary rotation angle interval (for example: 0.1 degree). Herein, the rotation angle interval may be arbitrarily changed, may be set with an interval except for the 0.1 degree interval, and may be used by setting according to a trade-off relation between a processing time and accuracy.

In addition, in order to improve a processing speed, an image rotation process may be performed by zooming in the CT image. For example, original information of 800*800*600 size may be processed by zooming in as 400*400*600, 400*400*300, 400*800*300, etc. In addition, in order to accurately calculate the rotation angle for the image correction, the image rotation process may be performed after applying a pre-process algorithm such as a noise removing filter, etc.

For example, the rotation angle section of the designated CT image for each cross-section may be set as (-10°~ 10°) for axial, (-9°~ 9°) for sagittal, and (-3°~ 3°) for coronal, and the above example corresponds to one example.

In addition, setting of the rotation angle may be used by parameterizing according to each device and considering an error range of the CT radiograph output data.

In addition, the image rotation may be performed by repeating an operation of the image data extractor 112b and the count map extractor 112c which will be described later while an angle is changed by the preset rotation angle interval (for example: 0.1 degree) along the axial direction, the sagittal direction, and coronal direction.

In addition, an order of image rotation may be arbitrarily selected, a method of finding a rotation angle by determining count maps for all directions may be possibly used, and a method of finding a rotation angle by sequentially determining count maps of the axial direction, the sagittal direction, and the coronal direction.

In addition, the image data extractor 112b extracts the image data of the ROI designated for each cross-section based on the lower jawline information (base point) extracted in the base point extractor 111 from the CT image rotated in the image rotator 112a. Herein, the ROI may be set with a size which can include both of the upper jaw and the lower jaw. Herein, the setting of the ROI may be adjusted other than the form of including both of the upper jaw and the lower jaw, since the ROI is a factor that may affect the performance of the device (or algorithm) according to the embodiment of the present invention.

In addition, the count map extractor 112c extracts the count map based on the designated threshold value by using the image data within the ROI extracted in the image data extractor 112b. Herein, the Formula g(x,y) for generating the count map is as the [Formula 9] below.

$$[Formula\ 9]$$

$$g(x,y) = \sum_{z=r1}^{r2} \sum_{y=0}^{h-1} \sum_{x=0}^{w-1} f(x,y,z)$$

Herein, r1 and r2 are height values of the set ROI.

In addition, the Formula f(x,y,z) for a count map generation condition is as the [Formula 10] below.

$$[Formula\ 10]$$

$$f(x,y,z) = \{1, \qquad t1 < I(x,y,z) < t2\}$$

Herein, the designated threshold value t1 may be 500, and t2 may be 1500, or may be set by varying according to the CT radiograph device or a radiograph environment.

Meanwhile, the setting unit 200 includes: a positional information setting unit 210 configured to set a positional coordinate of the X-ray source based on a 2D viewing direction of a cephalometric image with reference to a radiographing sequence information of the radiographing device; and an active section setting unit 220 setting an active image data section based on the positional coordinate set in the positional information setting unit 210.

Herein, the positional information setting unit 210 sets the positional coordinate of the X-ray source based on a positional coordinate of an anterior teeth occlusion surface with reference to the radiographing sequence information. For example, in the embodiment of the present invention, the most reasonable position of the X-ray source for the cephalometric image radiographing device may be set to be in a planar surface including the anterior teeth occlusion surface. In addition, considering a position of radiographing the cephalometric image, the position of the X-ray source may be properly set in an outside area of an 3D medical image.

A cephalometric image is reconstructed by setting the positional coordinate of the X-ray source as above, and performing a summation of image data of a designated section along an X-ray irradiation path based on the positional coordinate of the X-ray source. Herein, the X-ray irradiation path for the summation is determined based on the position of the X-ray source. The X-ray path may be determined by calculating where X-ray is generated at an X-ray source position and each voxel positions of a CT radiograph which the X-ray passes through considering radiographing with an actual cephalometric image radiograph device.

In addition, the active section setting unit 220 may set the entire section or a designated partial section of the CT image in a sagittal or coronal cross-sectional direction based on the X-ray beam irradiation path as a summation section (active image data section).

Meanwhile, the reconstructing unit 300 includes: an adjusting unit 310 changing a ratio of image data that is reflected on a reconstructing cephalometric image; and a medical image reconstructor 320 reconstructing a cephalometric image by performing a summation of "image data within the active image data section set in the active section setting unit 220" and which is adjusted in the adjusting unit 310.

Herein, the reconstructing unit 300 further includes an image size adjusting unit 330 adjusting a size of the cephalometric image reconstructed in the medical image reconstructor 320.

In addition, the reconstructing unit 300 further includes a medical image processor 340 performing image processing to the cephalometric image reconstructed in the medical image reconstructor 320.

Herein, the adjusting unit 310 generates and uses an opacity table. In other words, considering a characteristic of a CT number, the opacity table is generated by applying a gamma curve to an area with a specific threshold value or less. Herein, the opacity table is a kind of a look-up table, and is used as one method of adjusting a ratio of image data that is reflected on the reconstructing cephalometric image in the present invention. The Formula T(x) for generating the opacity table is as the [Formula 11] below.

[Formula 11]

$$T(x) = \sum_{x=0}^{s} f(x)$$

$$f(x) = \begin{cases} (x/(t-1))^g \times x, & x < t \\ x, & x \geq t \end{cases}$$

Herein, s means a size of the opacity table, and g means a gamma curve power. The threshold value t may be applied by being optimally calculated through an experiment. For example, when the threshold value t is 2000, the above value is one example that is set by considering a CT number.

In addition, among the image data of the CT image corrected in the geometry correction unit 100, the medical image reconstructor 320 reconstructs the cephalometric image by performing the summation of "the image data within the active image data section set in the active section setting unit 220" and which is adjusted in the adjusting unit 310.

Herein, when performing the summation, the opacity table may be applied to values of an active image data section. In addition, the values of the active image data section may be determined by adjusting a weight factor of summation information (image data) utilizing feature information (edge, gradient, etc.) Herein, considering a characteristic of the X-ray, the weight factor may use an attenuation coefficient, or may use an intensity value of a pixel within the summation section. Then, the summation is performed by applying the finally generated weight factor to a result value that the opacity table is applied.

The cephalometric image is reconstructed by applying the above process to all the X-ray path.

In addition, the medical image size adjusting unit 330 adjusts the size of the cephalometric image reconstructed in the CT information. In other words, the image size is adjusted by cutting a specific area to derive a result image similar to an image which is radiographed in the actual cephalometric image radiographing device or to remove unnecessary border parts. Herein, if adjusting of the image size is not necessary, the operation of the medical image size adjusting unit 330 may be omitted.

In addition, the medical image processor 340 improves image quality by performing a post processing to the reconstructed cephalometric image. In order to maximize the image quality improvement, a pre-processing process may be applied before the geometry correction unit 100.

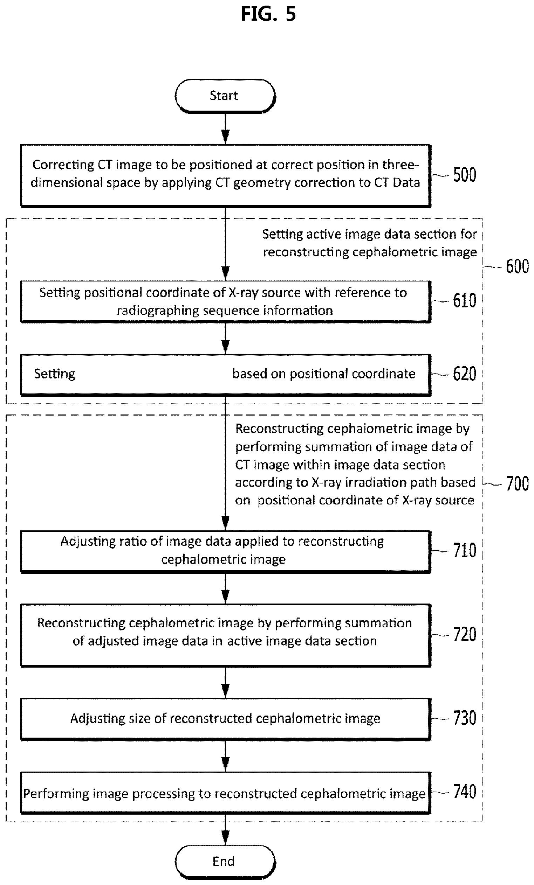

FIG. 5 is a flowchart of a medical image reconstruction method according to an embodiment of the present invention. The detailed embodiment thereof has been described in detail in the description of the medical image reconstruction device. Hereinafter, an operation process thereof will be briefly described.

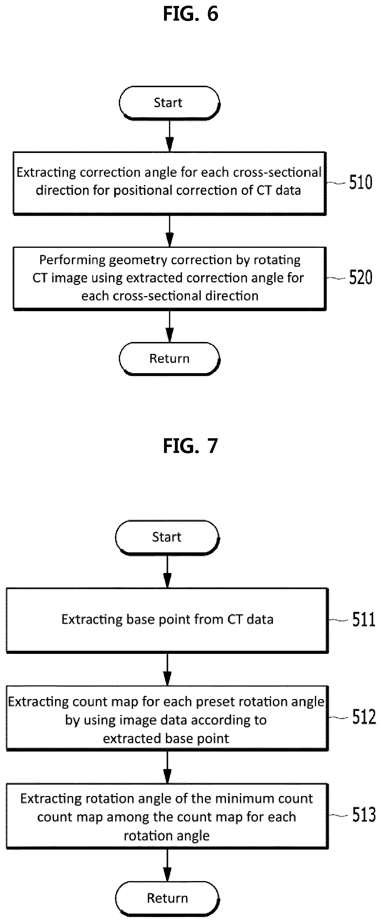

First, in step 500, the geometry correction unit 100 corrects so that a CT image is positioned at a correct position in a three-dimensional space by applying a CT geometry correction to CT data. The process of the geometry correction will be described later with reference to FIGS. 6 to 8.

Then, in step 600, the setting unit 200 sets an active image data section that will be used for reconstructing a cephalometric image. Herein, the step 600 of setting the active image data section includes: in step 610, setting, by the positional information setting unit 210, a positional coordinate of the X-ray source with reference to radiographing sequence information; and in step 620, setting, by the active section setting unit 220, an active image data section based on the positional coordinate set in the positional information setting unit 210.

Then, in step 700, the reconstructing unit 300 reconstructs the cephalometric image by performing a summation of "image data of the CT image corrected in the geometry correction unit 100" within the active image data section set in the setting unit 200 along an X-ray irradiation path based on the positional coordinate of the X-ray source set in the setting unit 200. Herein, the step 700 of reconstructing the cephalometric image includes: in step 710, adjusting, by the adjusting unit 310, a ratio of the image data that is used for the reconstructing cephalometric image; and in step 720, reconstructing, by the medical image reconstructor 320, the cephalometric image by performing the summation of "the image data within the active image data section set in the active section setting unit 220" and which is adjusted in the adjusting unit 310. In addition, the step 700 of reconstructing the cephalometric image further includes: in step 730, adjusting, by the image size adjusting unit 330, a size of the cephalometric image reconstructed in the medical image reconstructor 320. In addition, the step 700 of reconstructing the cephalometric image further includes: in step 740, performing, by the medical image processor 340, image processing to the cephalometric image reconstructed in the medical image reconstructor 320.

FIG. 6 is a detailed flowchart of the step 500 of the geometry correction according to the embodiment of the present invention.

First, in step 510, the correction angle extractor 110 extracts a correction angle for each cross-sectional direction to correct the position of the CT image by receiving the CT information. Herein, in order to extract the correction angle for each cross-section, a sub routine (correction angle extraction process of FIG. 7) may be performed for each cross-section.

Then, in step 520, the corrector 120 performs the geometry correction by rotating the CT image according to the correction angle for each cross-sectional direction extracted in the correction angle extractor 110.

FIG. 7 is a detailed flowchart of the step 510 of extracting the correction angle according to the embodiment of the present invention.

First, in step 511, the base point extractor 111 extracts base point in the CT information.

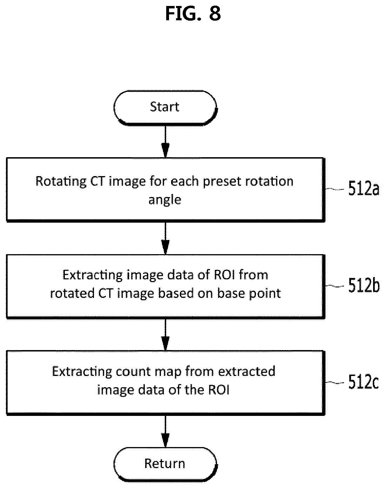

Then, in step 512, the rotation angle count map extractor 112 extracts a count map for preset each rotation angle by using image data according to the base point extracted in the base point extractor 111. Herein, in order to extract the count map for each rotation angle, a sub routine (rotation angle count map extraction process of FIG. 8) may be performed for each rotation angle.

Next, in step 513, the rotation angle extractor 113 extracts a rotation angle of the minimum count map among the count map for each rotation angle extracted in the rotation angle count map extractor 112.

FIG. 8 is a detailed flowchart of the step 512 of extracting the rotation angle count map according to the embodiment of the present invention.

First in step 512a, the image rotator 112a rotates the CT image for each preset rotation angle.

Then, in step 512b, the image data extractor 112b extracts image data of a ROI from the CT image rotated in the image rotator 112a based on the base point extracted in the base point extractor 111.

Next, in step 512c, the count map extractor 112c extracts a count map by using the image data of the ROI extracted in the image data extractor 112b.

Meanwhile, in the above embodiment, the CT image is corrected to be correctly positioned at the three-dimensional space by applying the CT geometry correction to the CT data.

Accordingly, when a CT image corrected according to the embodiment of the present invention is displayed through a viewer, regardless of radiographing target and posture, bilaterally symmetric organs such as eyes, ears, earlobes, etc., or feature points are permanently arranged on a horizontal surface.

In addition, in the above embodiment, a cephalometric image is generated by using a CT image and by using an automatic reconstruction method. Herein, in the embodiment of the present invention, a cephalometric image is generated by reconstructing image data of a specific section in a sagittal or coronal cross-sectional direction of a CT image based on an X-ray irradiation path.

Accordingly, when a cephalometric image according to the embodiment of the present invention is displayed, magnification ratios of the cephalometric image are different by positions along an X-ray irradiation path. As a simple example, in a cephalometric image of a sagittal cross-sectional direction, in other words, in a lateral direction, sizes of right/left eyes appear to be different, and it is similar to a cephalometric image using an actual cephalometric radiographing device.

A medical image reconstruction method in accordance with an example of the present invention may be implemented in the form of program instructions that can be executed by a variety of computer means, and may be stored in a computer-readable storage medium. The computer-readable storage medium may includes program instructions, and further a data file, and a data structure solely or in combination. The program instructions that are stored in the medium may be designed and constructed particularly for the present invention, or may be known and available to those skilled in the field of computer software. Examples of the computer-readable storage medium include magnetic media such as a hard disk, a floppy disk and a magnetic tape; optical media such as CD-ROM and a DVD; magneto-optical media such as a floptical disk; and hardware devices particularly configured to store and execute program instructions such as ROM, RAM, and flash memory. Examples of the program instructions include not only machine language code that is constructed by a compiler but also high-level language code that can be executed by a computer using an interpreter or the like. The above-described hardware components may be configured to act as one or more software modules that perform the operation of the present invention, and vice versa.

While the present invention has been described in conjunction with specific details, such as specific configuration elements, and limited examples and diagrams above, these are provided merely to help an overall understanding of the present invention, the present invention is not limited to these examples, and various modifications and variations can be made from the above description by those having ordinary knowledge in the art to which the present invention pertains.

The invention is defined by the appended claims.

## Claims

1. A device for reconstructing a medical cephalometric image, the device comprising:

    a geometry correction unit (100) configured to correct a geometry of three-dimensional medical image data by rotating a three-dimensional medical image so that at least one of jawline image data, teeth arrangement image data, ear plug image data, temporo-mandibular joint "TMJ" image data, and eye positional image data of the three-dimensional medical image data is arranged on a horizontal level;
    a setting unit (200) configured to set an active image data section in the three-dimensional medical image; and
    a reconstructing unit (300) configured to reconstruct the medical cephalometric image by performing a summation of the three-dimensional medical image data in the active image data section in a coronal direction or a sagittal direction,
    **characterized in that** the geometry correction unit (100) includes:

    a correction angle extractor (110) configured to extract a correction angle of the three-dimensional medical image for at least one cross-section of the coronal direction the sagittal direction and an axial direction by using a count map; and
    a corrector (120) configured to rotate the three-dimensional medical image by using the correction angle, wherein the correction angle extractor (110) includes:

        a base point extractor (111) configured to extract a base point in the three-dimensional medical image data, wherein the base point is at least one of jawline information, teeth arrangement information, ear plug information, temporo-mandibular joint "TMJ" information, and eye positional information;
        an image rotator (112a) configured to rotate the three-dimensional medical image with a plurality of predetermined rotation angles;
        a rotation angle count map extractor (112) configured to extract a count map informing about the number of pixels within a threshold value range according to the rotation angles, and further configured to overlap the three-dimensional medical image data of an ROI, a region of interest including the base point, to generate an overlapped image for each rotation angle,
        calculating the value of each pixel of the overlapped image, and
        calculating the number of pixels within the threshold value range; and
        a rotation angle extractor (113) configured to extract the rotation angle having the lowest number of pixels satisfying the threshold value range from the count map, as the correction angle for each cross-sectional direction.

2. The device of claim 1, wherein the setting unit (200) includes a positional information setting unit (210) configured to set positional coordinate of an X-ray source to be outside of the three-dimensional medical image, wherein the reconstructing unit (300) is configured to perform the summation of the three-dimensional medical image data according an X-ray irradiation path generated from the positional coordinate.

3. The device of claim 1, wherein the medical cephalometric image has different magnification ratios at different positions along the two-dimensional viewing direction.

4. A method of reconstructing a medical cephalometric image using a medical image reconstruction device of any of claims 1 to 3, the method comprising the steps of:

(a) correcting a geometry of three-dimensional medical image data by rotating a three-dimensional medical image so that at least one of jawline image data, teeth arrangement image data, ear plug image data, temporo-mandibular joint "TMJ" image data, and eye positional image data of the three-dimensional medical image data is arranged on a horizontal level;
(b) setting an active image data section in the three-dimensional medical image (600); and
(c) reconstructing the medical cephalometric image by performing a summation of the three-dimensional medical image data in the active image data section in a coronal direction or a sagittal direction (700),

characterized in that the step (a) includes the steps of:

(a1) extracting a correction angle of the three-dimensional medical image for at least one cross-section of the coronal direction, the sagittal direction and an axial direction by using a count map; and
(a2) rotating the three-dimensional medical image by using the correction angle,

wherein the step (a1) includes the steps of:

(a11) extracting a base point in the three-dimensional medical image data, wherein the base point is at least one of jawline information, teeth arrangement information, ear plug information, temporo-mandibular joint "TMJ" information, and eye positional information;
(a12) rotating the three-dimensional medical image data with a plurality of predetermined rotation angles; extracting a count map informing about the number of pixels within a threshold value range according to the rotation angles; overlapping the three-dimensional medical image of an ROI, a region of interest including the base point, to generate an overlapped image for each rotation angle, calculating the value of each pixel of the overlapped image, and calculating the number of pixels within the threshold value range;
(a13) extracting the rotation angle having the lowest number of pixels satisfying the threshold value range from the count map, as the correction angle for each cross-sectional direction.

5. The method of claim 4, further comprising: before the step (c), setting a positional coordinate of an X-ray source outside of the three-dimensional medical image, and the step (c) includes: performing the summation of the three-dimensional medical image data based on an X-ray irradiation path generated from the positional coordinate.

6. The method of claim 4, wherein the medical cephalometric image has different magnification ratios at different positions along the two-dimensional viewing direction.

7. A computer readable recording medium on which a program is recorded for implementing the method of any one of claims 4 to 6.

**Patentansprüche**

1. Vorrichtung zum Rekonstruieren eines medizinischen kephalometrischen Bildes, wobei die Vorrichtung umfasst:

eine Geometriekorrektureinheit (100), die so konfiguriert ist, dass sie eine Geometrie dreidimensionaler medizinischer Bilddaten korrigiert, indem sie ein dreidimensionales medizinisches Bild so dreht, dass mindestens eines von Kieferlinienbilddaten, Zahnanordnungsbilddaten, Ohrstöpselbilddaten, Temporo-Mandibulargelenk-"TMJ"-Bilddaten und Augenpositionsbilddaten der dreidimensionalen medizinischen Bilddaten auf einer horizontalen Ebene angeordnet ist;

eine Einstelleinheit (200), die konfiguriert ist, einen aktiven Bilddatenabschnitt in dem dreidimensionalen medizinischen Bild einzustellen; und

eine Rekonstruktionseinheit (300), die so konfiguriert ist, dass sie das medizinische kephalometrische Bild rekonstruiert, indem sie eine Summierung der dreidimensionalen medizinischen Bilddaten in dem aktiven Bilddatenabschnitt in einer koronalen Richtung oder einer sagittalen Richtung durchführt,

**dadurch gekennzeichnet, dass** die Geometriekorrektureinheit (100) Folgendes umfasst:

einen Korrekturwinkel-Extraktor (110), der konfiguriert ist, um einen Korrekturwinkel des dreidimensionalen medizinischen Bildes für mindestens einen Querschnitt der koronalen Richtung, der sagittalen Richtung und einer axialen Richtung unter Verwendung einer Zählkarte zu extrahieren; und

einen Korrektor (120), der konfiguriert ist, um das dreidimensionale medizinische Bild unter Verwendung des Korrekturwinkels zu drehen,

wobei der Korrekturwinkel-Extraktor (110) umfasst

einen Basispunktextraktor (111), der so konfiguriert ist, dass er einen Basispunkt in den dreidimensionalen medizinischen Bilddaten extrahiert, wobei der Basispunkt mindestens eine der folgenden Informationen ist: Kieferlinieninformation, Zahnanordnungsinformation, Ohrstöpselinformation, Temporo-Mandibulargelenk-"TMJ"-Information und Augenpositionsinformation;

einen Bildrotator (112a), der konfiguriert ist, um das dreidimensionale medizinische Bild mit einer Vielzahl von vorbestimmten Rotationswinkeln zu rotieren einen Rotationswinkel-Zählkarten-Extraktor (112), der konfiguriert ist, um eine Zählkarte zu extrahieren, die über die Anzahl von Pixeln innerhalb eines Schwellenwertbereichs gemäß den Rotationswinkeln informiert, und der ferner konfiguriert ist, um Überlappen der dreidimensionalen medizinischen Bilddaten einer ROI, Region von Interesse, die den Basispunkt enthält, um ein überlapptes Bild für jeden Rotationswinkel zu erzeugen,

Berechnen des Wertes eines jeden Pixels des überlappten Bildes, und

Berechnen der Anzahl von Pixeln innerhalb des Schwellenwertbereichs; und

einen Drehwinkel-Extraktor (113), der so konfiguriert ist, dass er den Drehwinkel mit der geringsten Anzahl von Pixeln, die den Schwellenwertbereich erfüllen, aus der Zählkarte als den Korrekturwinkel für jede Querschnittsrichtung extrahiert.

2. Vorrichtung nach Anspruch 1, wobei die Einstelleinheit (200) eine Positionsinformations-Einstelleinheit (210) enthält, die so konfiguriert ist, dass sie eine Positionskoordinate einer Röntgenquelle einstellt, die außerhalb des dreidimensionalen medizinischen Bildes liegen soll,

wobei die Rekonstruktionseinheit (300) so konfiguriert ist, dass sie die Summierung der dreidimensionalen medizinischen Bilddaten gemäß einem aus der Positionskoordinate erzeugten Röntgenstrahlungspfad durchführt.

3. Vorrichtung nach Anspruch 1, wobei das kephalometrische medizinische Bild unterschiedliche Vergrößerungsverhältnisse an unterschiedlichen Positionen entlang der zweidimensionalen Betrachtungsrichtung aufweist.

4. Verfahren zum Rekonstruieren eines medizinischen kephalometrischen Bildes unter Verwendung einer medizinischen Bildrekonstruktionsvorrichtung nach einem der Ansprüche 1 bis 3, wobei das Verfahren die folgenden Schritte umfasst:

(a) Korrigieren einer Geometrie dreidimensionaler medizinischer Bilddaten durch Drehen eines dreidimensionalen medizinischen Bildes, so dass mindestens eines von Kieferlinien-Bilddaten, Zahnanordnungs-Bilddaten und Augenpositions-Bilddaten der dreidimensionalen medizinischen Bilddaten auf einer horizontalen Ebene angeordnet ist;

(b) Einstellen eines aktiven Bilddatenabschnitts in dem dreidimensionalen medizinischen Bild (600); und

(c) Rekonstruieren des kephalometrischen medizinischen Bildes durch Durchführen einer Summierung der dreidimensionalen medizinischen Bilddaten in dem aktiven Bilddatenabschnitt in einer koronalen Richtung oder einer sagittalen Richtung (700) **dadurch gekennzeichnet, dass** der Schritt (a) die folgenden Schritte umfasst:

(a1) Extrahieren eines Korrekturwinkels des dreidimensionalen medizinischen Bildes für mindestens einen Querschnitt der koronalen Richtung, der sagittalen Richtung und einer axialen Richtung unter Verwendung einer Zählkarte; und

(a2) Drehen des dreidimensionalen medizinischen Bildes unter Verwendung des Korrekturwinkels, wobei der Schritt (a1) die folgenden Schritte umfasst:

(a11) Extrahieren eines Basispunktes in den dreidimensionalen medizinischen Bilddaten, wobei der Basispunkt mindestens eine der folgenden Informationen ist: Kieferlinieninformation, Zahnanordnungsinformation, Ohrstöpselinformation, Kiefergelenk-"TMJ"-Information und Augenpositionsinformation;

(a12) Drehen der dreidimensionalen medizinischen Bilddaten mit einer Vielzahl von vorbestimmten Drehwinkeln; Extrahieren einer Zählkarte, die über die Anzahl von Pixeln innerhalb eines Schwellenwertbereichs gemäß den Drehwinkeln informiert;

Überlappen des dreidimensionalen medizinischen Bildes einer ROI, einer Region von Interesse, die den Basispunkt enthält, um ein überlapptes Bild für jeden Drehwinkel zu erzeugen, Berechnen des Wertes jedes Pixels des überlappten Bildes und

Berechnen der Anzahl von Pixeln innerhalb des Schwellenwertbereichs;

(a13) Extrahieren des Drehwinkels mit der geringsten Anzahl von Pixeln, die den Schwellenwertbereich erfüllen, aus der Zählkarte als Korrekturwinkel für jede Querschnittsrichtung.

5. Verfahren nach Anspruch 4, ferner umfassend: vor dem Schritt (c) Festlegen einer Positionskoordinate einer Röntgenquelle außerhalb des dreidimensionalen medizinischen Bildes, und der Schritt (c) umfasst: Durchführen der Summierung der dreidimensionalen medizinischen Bilddaten auf der Grundlage eines aus der Positionskoordinate erzeugten Röntgenbestrahlungspfades.

6. Verfahren nach Anspruch 4, wobei das medizinische kephalometrische Bild an verschiedenen Positionen entlang der zweidimensionalen Betrachtungsrichtung unterschiedliche Vergrößerungsverhältnisse aufweist.

7. Computerlesbares Aufzeichnungsmedium, auf dem ein Programm zur Implementierung des Verfahrens nach einem der Ansprüche 4 bis 6 aufgezeichnet ist.

**Revendications**

1. Dispositif pour reconstruire une image céphalométrique médicale, le dispositif comprenant :

une unité de correction de géométrie (100) configurée pour corriger une géométrie de données d'image médicale tridimensionnelle en faisant tourner une image médicale tridimensionnelle de sorte qu'au moins l'une des données d'image de ligne de mâchoire, des données d'image d'agencement de dents, des données d'image de bouchon d'oreille, des données d'image d'articulation temporo-mandibulaire "TMJ",

et des données d'image de position d'oeil des données d'image médicale tridimensionnelle soit disposée sur un niveau horizontal ;

une unité de réglage (200) configurée pour régler une section de données d'image active dans l'image médicale tridimensionnelle ; et

une unité de reconstruction (300) configurée pour reconstruire l'image médicale céphalométrique en effectuant une sommation des données d'image médicale tridimensionnelle dans la section de données d'image active dans une direction coronale ou une direction sagittale,

**caractérisé en ce que** l'unité de correction géométrique (100) comprend :

un extracteur d'angle de correction (110) configuré pour extraire un angle de correction de l'image médicale tridimensionnelle pour au moins une section transversale de la direction coronale, de la direction sagittale et d'une direction axiale en utilisant une carte de comptage ; et

un correcteur (120) configuré pour faire tourner l'image médicale tridimensionnelle en utilisant l'angle de correction,

dans lequel l'extracteur d'angle de correction (110) comprend :

un extracteur de point de base (111) configuré pour extraire un point de base dans les données d'image médicale tridimensionnelle, dans lequel le point de base est au moins l'une des informations de ligne de mâchoire, des informations d'agencement de dents, des informations de bouchon d'oreille, des informations d'articulation temporo-mandibulaire "TMJ", et des informations de position d'oeil ;

un rotateur d'image (112a) configuré pour faire tourner l'image médicale tridimensionnelle avec une pluralité d'angles de rotation prédéterminés ;

un extracteur de carte de comptage d'angle de rotation (112) configuré pour extraire une carte de comptage fournissant des informations sur le nombre de pixels dans une plage de valeurs de seuil selon les angles de rotation, et configuré en outre pour superposer les données d'image médicale tridimensionnelle d'une région d'intérêt (ROI) comprenant le point de base, pour générer une image superposée pour chaque angle de rotation, calculer la valeur de chaque pixel de l'image superposée, et

calculer le nombre de pixels dans la plage de valeurs de seuil ; et

un extracteur d'angle de rotation (113) configuré pour extraire l'angle de rotation ayant le plus petit nombre de pixels satisfaisant la plage de valeurs de seuil à partir de la carte de comptage, comme angle de correction pour chaque direction de section transversale.

2. Dispositif de la revendication 1, dans lequel l'unité de réglage (200) comprend une unité de réglage d'informations de position (210) configurée pour régler une coordonnée de position d'une source de rayons X devant être à l'extérieur de l'image médicale tridimensionnelle,

dans lequel l'unité de reconstruction (300) est configurée pour effectuer la sommation des données d'image médicale tridimensionnelle selon un chemin d'irradiation de rayons X généré à partir de la coordonnée positionnelle.

3. Dispositif de la revendication 1, dans lequel l'image médicale céphalométrique présente différents rapports de grossissement à différentes positions le long de la direction de visualisation bidimensionnelle.

4. Procédé de reconstruction d'une image céphalométrique médicale utilisant un dispositif de reconstruction d'image médicale de l'une quelconque des revendications 1 à 3, le procédé comprenant les étapes consistant à :

(a) corriger une géométrie de données d'image médicale tridimensionnelle en faisant tourner une image médicale tridimensionnelle de sorte qu'au moins l'une des données d'image de ligne de mâchoire, des données d'image d'agencement de dents et des données d'image de position d'oeil des données d'image médicale tridimensionnelle soit disposée sur un niveau horizontal ;

(b) définir une section de données d'image active dans l'image médicale tridimensionnelle (600) ; et

(c) reconstruire l'image médicale céphalométrique en effectuant une sommation des données d'image médicale tridimensionnelle dans la section de données d'image active dans une direction coronale ou une direction sagittale (700) **caractérisé en ce que** l'étape (a) comprend les étapes consistant à :

(a1) extraire un angle de correction de l'image médicale tridimensionnelle pour au moins une section transversale de la direction coronale, de la direction sagittale et

d'une direction axiale en utilisant une carte de comptage ; et

(a2) faire tourner l'image médicale tridimensionnelle en utilisant l'angle de correction,

dans lequel l'étape (a1) comprend les étapes consistant à :

(a11) extraire un point de base dans les données d'image médicale tridimensionnelle, dans lequel le point de base est au moins une information parmi une information de ligne de mâchoire, une information de disposition de dents, une information de bouchon d'oreille, une information d'articulation temporo-mandibulaire "ATM", et une information de position d'oeil ;

(a12) faire tourner les données d'image médicale tridimensionnelle avec une pluralité d'angles de rotation prédéterminés ; extraire une carte de comptage informant du nombre de pixels dans une plage de valeurs de seuil selon les angles de rotation ;

superposer l'image médicale tridimensionnelle d'un ROI, une région d'intérêt comprenant le point de base, pour générer une image superposée pour chaque angle de rotation, calculer la valeur de chaque pixel de l'image superposée, et calculer le nombre de pixels dans la plage de valeurs de seuil ;

(a13) extraire de la carte de comptage l'angle de rotation ayant le plus petit nombre de pixels satisfaisant la plage de valeurs de seuil, en tant qu'angle de correction pour chaque direction de section transversale.

5. Procédé de la revendication 4, comprenant en outre : avant l'étape (c), le réglage d'une coordonnée de position d'une source de rayons X à l'extérieur de l'image médicale tridimensionnelle, et l'étape (c) comprend : l'exécution de la sommation des données d'image médicale tridimensionnelle sur la base d'un trajet d'irradiation de rayons X généré à partir de la coordonnée de position.

6. Procédé de la revendication 4, dans lequel l'image céphalométrique médicale présente différents rapports de grossissement à différentes positions le long de la direction de visualisation bidimensionnelle.

7. Support d'enregistrement lisible par ordinateur sur lequel un programme est enregistré pour mettre en œuvre le procédé de l'une quelconque des revendications 4 à 6.

**FIG. 1**

CT Data

↓

| Geometry Correction Unit | — 100 |

↓

Setting Unit — 200

| Positional Information Setting Unit | — 210 |

↓

| Active Data Setting Unit | — 220 |

Reconsctructing Unit — 300

↓

| Adjusting Unit | — 310 |

↓

| Medical Image Reconstructor | — 320 |

↓

| Medical Image Size Adjusting Unit | — 330 |

↓

| Medical Image Processor | — 340 |

↓

Reconstructed Cephalometric Image

# FIG. 2

CT Data

┌ ─ ─ ─ ─ ─ ─ ─ ─ ─ ─ ─ ─ ─ ─ ─ ─ ─ ─ ┐
│                          Geometry Correction Unit ⌐∼ 100
│  ┌─────────────────────────────────┐
│  │      Correction Angle Extractor      │∼ 110
│  └─────────────────────────────────┘
│  ┌─────────────────────────────────┐
│  │             Corrector              │∼ 120
│  └─────────────────────────────────┘
└ ─ ─ ─ ─ ─ ─ ─ ─ ─ ─ ─ ─ ─ ─ ─ ─ ─ ─ ┘

Geometrically Corrected Medical Image Data

# FIG. 3

CT Data                                    110

┌ ─ ─ ─ ─ ─ ─ ─ ─ ─ ─ ─ ─ ─ ─ ─ ─ ─ ─ ┐
│                          Correction Angle Extractor
│  ┌─────────────────────────────────┐
│  │        Base Point Extractor         │∼ 111
│  └─────────────────────────────────┘
│  ┌─────────────────────────────────┐
│  │  Rotation Angle Count Map Extractor   │∼ 112
│  └─────────────────────────────────┘
│  ┌─────────────────────────────────┐
│  │      Rotation Angle Extractor        │∼ 113
│  └─────────────────────────────────┘
└ ─ ─ ─ ─ ─ ─ ─ ─ ─ ─ ─ ─ ─ ─ ─ ─ ─ ─ ┘

Rotation Angle (Correction Angle)

# FIG. 4

Base Point

112

Rotation Angle Count Map Extractor

Image Rotator — 112a

Image Data Extractor — 112b

Count Map Extractor — 112c

Count Map

# FIG. 5

```
┌─────────────┐
│    Start    │
└─────────────┘
       │
       ▼
┌──────────────────────────────────────────────────────────┐
│ Correcting CT image to be positioned at correct position  │  ~ 500
│ in three-dimensional space by applying CT geometry        │
│ correction to CT Data                                      │
└──────────────────────────────────────────────────────────┘
       │
┌ ─ ─ ─ ─ ─ ─ ─ ─ ─ ─ ─ ─ ─ ─ ─ ─ ─ ─ ─ ─ ─ ─ ─ ─ ─ ─ ─ ┐
│                   Setting active image data section for   │
│                   reconstructing cephalometric image       │  ~ 600
│       ▼                                                    │
│ ┌────────────────────────────────────────────────────┐   │
│ │ Setting positional coordinate of X-ray source with  │   │  ~ 610
│ │ reference to radiographing sequence information      │   │
│ └────────────────────────────────────────────────────┘   │
│       │                                                    │
│       ▼                                                    │
│ ┌────────────────────────────────────────────────────┐   │
│ │ Setting            based on positional coordinate    │   │  ~ 620
│ └────────────────────────────────────────────────────┘   │
└ ─ ─ ─ ─ ─ ─ ─ ─ ─ ─ ─ ─ ─ ─ ─ ─ ─ ─ ─ ─ ─ ─ ─ ─ ─ ─ ─ ┘
       │
┌ ─ ─ ─ ─ ─ ─ ─ ─ ─ ─ ─ ─ ─ ─ ─ ─ ─ ─ ─ ─ ─ ─ ─ ─ ─ ─ ─ ┐
│             Reconstructing cephalometric image by          │
│             performing summation of image data of CT       │
│             image within image data section according to   │  ~ 700
│             X-ray irradiation path based on positional     │
│             coordinate of X-ray source                     │
│       ▼                                                    │
│ ┌────────────────────────────────────────────────────┐   │
│ │ Adjusting ratio of image data applied to             │   │  ~ 710
│ │ reconstructing cephalometric image                   │   │
│ └────────────────────────────────────────────────────┘   │
│       │                                                    │
│       ▼                                                    │
│ ┌────────────────────────────────────────────────────┐   │
│ │ Reconstructing cephalometric image by performing     │   │  ~ 720
│ │ summation of adjusted image data in active image     │   │
│ │ data section                                         │   │
│ └────────────────────────────────────────────────────┘   │
│       │                                                    │
│       ▼                                                    │
│ ┌────────────────────────────────────────────────────┐   │
│ │ Adjusting size of reconstructed cephalometric image  │   │  ~ 730
│ └────────────────────────────────────────────────────┘   │
│       │                                                    │
│       ▼                                                    │
│ ┌────────────────────────────────────────────────────┐   │
│ │ Performing image processing to reconstructed         │   │  ~ 740
│ │ cephalometric image                                  │   │
│ └────────────────────────────────────────────────────┘   │
└ ─ ─ ─ ─ ─ ─ ─ ─ ─ ─ ─ ─ ─ ─ ─ ─ ─ ─ ─ ─ ─ ─ ─ ─ ─ ─ ─ ┘
       │
       ▼
┌─────────────┐
│     End     │
└─────────────┘
```

# FIG. 6

Start

Extracting correction angle for each cross-sectional direction for positional correction of CT data ~ 510

Performing geometry correction by rotating CT image using extracted correction angle for each cross-sectional direction ~ 520

Return

# FIG. 7

Start

Extracting base point from CT data ~ 511

Extracting count map for each preset rotation angle by using image data according to extracted base point ~ 512

Extracting rotation angle of the minimum count count map among the count map for each rotation angle ~ 513

Return

# FIG. 8

```
            ( Start )
                |
                v
+-------------------------------------+
| Rotating CT image for each preset   |  ~ 512a
| rotation angle                      |
+-------------------------------------+
                |
                v
+-------------------------------------+
| Extracting image data of ROI from   |  ~ 512b
| rotated CT image based on base point|
+-------------------------------------+
                |
                v
+-------------------------------------+
| Extracting count map from extracted |  ~ 512c
| image data of the ROI               |
+-------------------------------------+
                |
                v
            ( Return )
```

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020130138612

**Non-patent literature cited in the description**

- **FARMAN et al.** Development of imaging selection criteria and procedures should precede computed tomographie. *American Journal of Orthondontics and Dentofacial Orthope,* 01 August 2006, vol. 130 (2), ISSN 0889-5406, 257-265